(19) Europäisches Patentamt
European Patent Office
Office européen des brevets

(11) **EP 2 238 900 B1**

(12) **EUROPEAN PATENT SPECIFICATION**

(45) Date of publication and mention
of the grant of the patent:
**28.11.2012 Bulletin 2012/48**

(51) Int Cl.:
***A61B 5/053*** *(2006.01)*　　　***A61B 5/107*** *(2006.01)*

(21) Application number: **10156726.1**

(22) Date of filing: **17.03.2010**

(54) **Subcutaneous fat thickness measurement apparatus**

Subkutane Fettdickenmessvorrichtung

Appareil de mesure de l'épaisseur de gras sous-cutané

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **07.04.2009 JP 2009092907
05.03.2010 JP 2010048954**

(43) Date of publication of application:
**13.10.2010 Bulletin 2010/41**

(73) Proprietor: **Tanita Corporation
Tokyo 174-8630 (JP)**

(72) Inventor: **Kasahara, Yasuhiro
Itabashi-ku, Tokyo 174-8630 (JP)**

(74) Representative: **Haley, Stephen
Gill Jennings & Every LLP
The Broadgate Tower
20 Primrose Street
London EC2A 2ES (GB)**

(56) References cited:
**EP-A1- 1 224 908　　EP-A1- 1 512 371
EP-A1- 1 584 290　　EP-A1- 1 621 131
US-A1- 2006 100 532**

## Description

The present invention relates to a subcutaneous fat thickness measurement apparatus for measuring the thickness of subcutaneous fat of a human body.

Conventionally, there is known a technique for measuring subcutaneous fat of a human body based on impedance determined by bringing hands and feet into contact with measurement electrodes (for example, refer to Japanese Patent Application Laid-Open Publication No. 2001-178697, hereinafter referred to as JP 2001-178697).

However, in a technique such as is disclosed in JP 2001-178697, only impedance is used to determine subcutaneous fat thickness. Therefore, it is not possible to acquire information only on fat. This is because a measured value of impedance can vary depending on the state of the muscles lying beneath the fat, and it is therefore difficult to accurately measure subcutaneous fat thickness.

US-A-2006-100532 discloses a subcutaneous fat thickness measurement apparatus with measurement electrodes.

In consideration of the above, the present invention has, as an object, to measure subcutaneous fat thickness with a high degree of accuracy.

In order to solve the above-described problem, the present invention provides a subcutaneous fat thickness measurement apparatus comprising:

measurement electrodes including a first current supply electrode, a second current supply electrode, a first voltage detection electrode, and a second voltage detection electrode, for use in measurement by contact with the body of a human subject;

an electric current generator for outputting alternating current that flows between the first current supply electrode and the second current supply electrode;

a voltage measurer for measuring voltage between the first voltage detection electrode and the second voltage detection electrode, the first voltage detection electrode located adjacent to the first current supply electrode and the second voltage detection electrode located adjacent to the second current supply electrode; and

a subcutaneous fat thickness measurer for obtaining subcutaneous fat thickness of a portion of the body between the first current supply electrode and the second current supply electrode that are in contact with the body based on the phase difference between electric current and voltage, the electric current flowing in a current pathway from one of the first current supply electrode and the second current supply electrode via the human body to the other of the first current supply electrode and the second current supply electrode when the measurement electrodes are in contact with the human body and the voltage being measured by the voltage measurer, characterized in that:

the subcutaneous fat thickness measurer is adapted to obtain a ratio between reactance and resistance obtained from impedance and the phase difference, to determine subcutaneous fat thickness corresponding to the obtained value of ratio, the impedance being calculated from the electric current flowing in the current pathway and the voltage measured by the voltage measurer.

According to the present invention there is further provided a subcutaneous fat thickness measurement apparatus according to claim 1, wherein; subcutaneous fat thickness obtained in the subcutaneous fat thickness measurer results in a greater value as a proportion of the reactance in the resistance is smaller, and in a smaller value as the proportion of the reactance in the resistance is greater.

In the present invention, "to measure" means to take a measurement or to estimate by calculation using at least one of measurement results and stored information. "To obtain" means to perform calculation or to estimate by calculation using at least one of measurement results and stored information. The stored information includes body-specific information input by a user, such as sex, age, and height of a human subject.

In this mode, focusing on the difference between the fat layer and the muscle layer in the phase difference generated, subcutaneous fat thickness of a portion of a human body with which the measurement electrodes are in contact is obtained based on the phase difference between electric current and voltage, the electric current flowing in a current pathway from one of the first current supply electrode and the second current supply electrode via the human body to the other of the first current supply electrode and the second current supply electrode when the measurement electrodes are in contact with the human body and the voltage being measured by the voltage measurer. More specifically, the subcutaneous fat thickness measurer obtains the subcutaneous fat thickness based on reactance (the imaginary part of impedance) and resistance (the real part of impedance) obtained from impedance and the phase difference. The impedance is calculated from current that flows in the current pathway and voltage measured by the voltage measurer.

More specifically, the muscle layer has a property of easily causing a phase difference, whereas the fat layer has a property in which it is difficult to cause the phase difference. Therefore, the greater the subcutaneous fat thickness, the more dominant the property of the fat layer, and the smaller the phase difference. On the other hand, the smaller the subcutaneous fat thickness, the more dominant the property of the muscle layer, and the greater the phase difference. As a result, the greater the subcutaneous fat thickness, the smaller the proportion of reactance in resistance; the smaller the subcutaneous fat thickness, the larger the proportion of reactance in resistance. Using this relationship, subcutaneous fat thickness measurer obtains the ratio between reactance and resistance, to determine subcutaneous fat thickness corresponding to a value of the ratio. Therefore, the present invention has an advantage in that the subcutaneous fat thickness can be measured with a high degree of accuracy.

With the invention, the distance between the first current supply electrode and the second current supply electrode can be reduced in comparison with a mode in which the first voltage detection electrode and the second voltage detection electrode are sandwiched between the first current supply electrode and the second current supply electrode. Generally, the amount of fat varies depending on the part of the human body (i.e., the subcutaneous fat thickness varies). Therefore, if the distance between the first current supply electrode and the second current supply electrode is large, the apparatus is susceptible to inadvertent measurement errors. According to the present invention, because the distance between the first current supply electrode and the second current supply electrode can be reduced, subcutaneous fat thickness can be measured with pinpoint localization. There is an advantage in that the degree of accuracy can be enhanced.

IN THE DRAWINGS

Fig. 1 is a diagram showing an external view of a subcutaneous fat thickness measurement apparatus according to an embodiment of the present invention.
Fig. 2 is a block diagram showing a detailed configuration of the subcutaneous fat thickness measurement apparatus according to the embodiment.
Fig. 3 is a flowchart showing an operation of a subcutaneous fat thickness measurement apparatus according to the embodiment.
Fig. 4 is a schematic diagram showing the tissue of a human body.
Fig. 5 is a diagram showing an equivalent circuit of the tissue of a human body.
Fig. 6 is a schematic diagram showing how an alternating current flows through a human body.
Fig. 7 is an equivalent circuit showing the muscle layer and the fat layer.
Fig. 8 is a correlation diagram showing the relationship between subcutaneous fat thickness estimated by a method of the present embodiment and fat thickness measured by the ultrasonic wave.
Fig. 9 is a correlation diagram showing the relationship between visceral fat area estimated by a method of the present embodiment and visceral fat area measured by a CT (Computed Tomography) scanning.
Fig. 10 is a correlation diagram showing the relationship between visceral fat mass estimated by a method of the present embodiment and visceral fat mass measured by CT scanning.
Fig. 11 is a correlation diagram showing subcutaneous fat area estimated by a method of the present embodiment and subcutaneous fat area measured by CT scanning.
Fig. 12 is a correlation diagram showing the relationship between subcutaneous fat mass estimated by a method of the present embodiment and subcutaneous fat mass measured by CT scanning.
Fig. 13 is a diagram showing an external view of a subcutaneous fat thickness measurement apparatus according to a modification of the present invention.
Fig. 14 is a block diagram showing a detailed configuration of the subcutaneous fat thickness measurement apparatus according to the modification.
Fig. 15 is a flowchart showing an operation of the subcutaneous fat thickness measurement apparatus according to the modification.
Fig. 16 is a diagram showing the relationship among impedance, reactance, resistance, and phase difference.
Fig. 17 is a diagram showing the relationship between phase difference and subcutaneous fat thickness.
Fig. 18 is a diagram showing the relationship between the ratio between two impedances for which frequencies are different from each other and the ratio between reactance and resistance.
Fig. 19 is a diagram showing the relationship between the ratio between two impedances for which frequencies are different from each other and subcutaneous fat thickness.

DETAILED DESCRIPTION OF PREFERRED EMBODIMENTS

A: Configuration

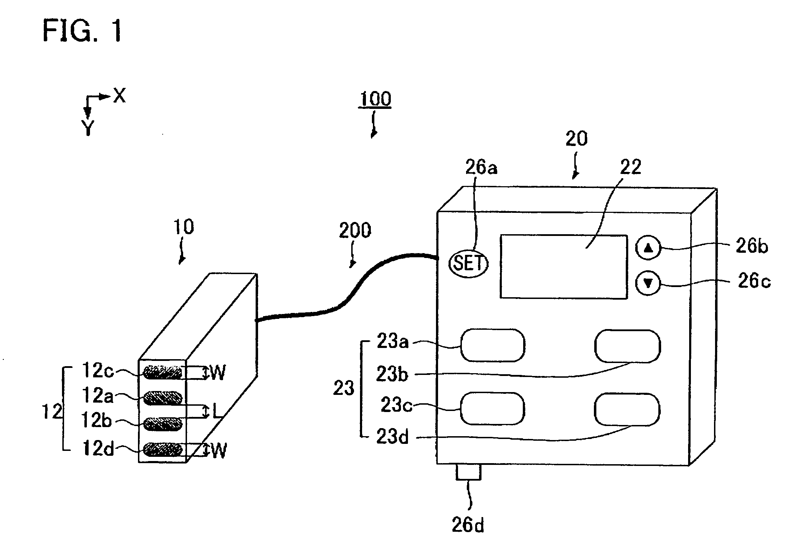

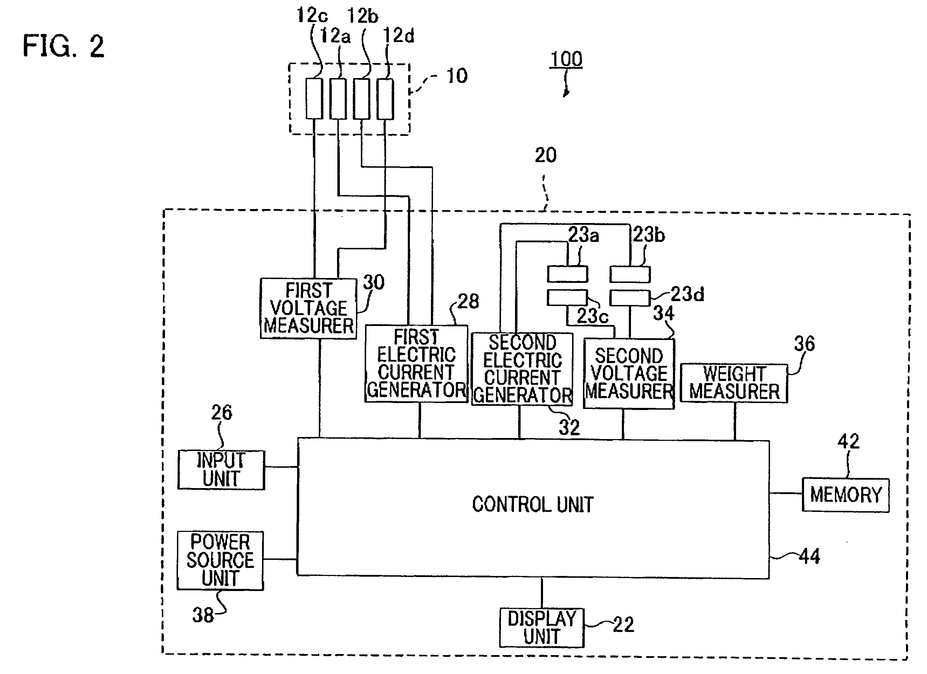

Fig. 1 is a diagram showing an external view of a subcutaneous fat thickness measurement apparatus 100 according to the present embodiment, and Fig. 2 is a block diagram showing a detailed configuration of subcutaneous fat thickness measurement apparatus 100 according to the present embodiment. Subcutaneous fat thickness measurement apparatus 100 according to the present embodiment not only has a function of measuring the subcutaneous fat thickness of a human subject but also has a function of measuring, by a publically known method, obesity-related information such as weight, body fat percentage, and body fat mass. As shown in Fig. 1, subcutaneous fat thickness measurement apparatus 100 has a handheld unit 10 and a platform unit 20. Handheld unit 10 is connected to platform unit 20 via a cable 200, and arranged in the forefront face of handheld unit 10 are first measurement electrodes 12 (12a, 12b,12c,12d) for use in measuring subcutaneous fat thickness by bringing them into contact with a human body.

First measurement electrodes 12 include a first current supply electrode 12a, a second current supply electrode 12b, a first voltage detection electrode 12c, and a second voltage detection electrode 12d. First current supply electrode 12a and second current supply electrode 12b are arranged sandwiched between first voltage detection electrode 12c and second voltage detection electrode 12d. Furthermore, first voltage detection electrode 12c is adjacent to first current supply electrode 12a, and second voltage detection electrode 12d is adjacent to second current supply electrode 12b. More specifically, first measurement electrodes 12 (12a, 12b, 12c, 12d) are aligned along a Y direction of the figure (first direction) in the forefront face of handheld unit 10; first voltage detection electrode 12c is arranged, adjacent to first current supply electrode 12a, on the negative side in the Y direction in relation thereto; and second voltage detection electrode 12d is arranged adjacent to second current supply electrode 12b on the positive side in the Y direction in relation thereto.

Distance L between first current supply electrode 12a and second current supply electrode 12b in the Y direction is set in such a way that the distance L is smaller than the sum of the width W of first voltage detection electrode 12c in the Y direction and the width W of second voltage detection electrode 12d in the Y direction. In the present embodiment, the size of each measurement electrode is set identical to one another, and therefore, the width W of first voltage detection electrode 12c in the Y direction and the width W of second voltage detection electrode 12d in the Y direction are the same (i.e., L<2W), Furthermore, in the present embodiment, the distance between two adjacent measurement electrodes in the Y direction is set identical to the distance between another two adjacent measurement electrodes, and the value thereof is equal to the width W of a measurement electrode in the Y direction (i.e., L = W). In this example, the value of the width W of a measurement electrode in the Y direction is set to 5 mm.

As shown in Figs. 1 and 2, platform unit 20 has, on the exterior thereof, a display unit 22, an input unit 26 (26a, 26b,26c,26d), and second measurement electrodes 23 (23a,23b,23c,23d). Second measurement electrodes 23 are electrodes for use in measuring body fat percentage of a human subject by bringing them into touch with the soles of the feet of the human subject. Second measurement electrodes 23 include a third current supply electrode 23a, a fourth current supply electrode 23b, a third voltage detection electrode 23c, and a fourth voltage detection electrode 23d. Third current supply electrode 23a and fourth current supply electrode 23b are arranged apart from each other in the X direction. More specifically, third current supply electrode 23a is arranged so as to correspond to a place on which the sole of the left foot of a human subject is placed; and fourth current supply electrode 23b is arranged so as to correspond to a place on which the sole of the right foot of a human subject is placed. Furthermore, third voltage detection electrode 23c is arranged, adjacent to third current supply electrode 23a, on the positive side in the Y direction in relation thereto, and is arranged corresponding to a place on which the sole of the left foot of a human subject is placed. Fourth voltage detection electrode 23d is arranged, adjacent to fourth current supply electrode 23b, on the positive side in Y direction, and arranged corresponding to a place on which the sole of the right foot of a human subject is placed.

Input unit 26 (26a,26b,26c,26d) includes a setting key 26a, an UP key 26b, a DOWN key 26c, and a start key 26d. UP key 26b and DOWN key 26c are used for selecting information and for switching numerals, and setting key 26a sets the selected information and the switched numerals. Start key 26d is a means for causing power supply to be started, the power being supplied to platform unit 20 for a series of measurements performed therein. Description will be given below of a detailed configuration of platform unit 20.

As shown in Fig. 2, platform unit 20 further has a first electric current generator 28, a first voltage measurer 30, a second electric current generator 32, a second voltage measurer 34, a weight measurer 36, a power source unit 38, a memory 42, and a controller 44.

First electric current generator 28 is a means for outputting an alternating current that flows between first current supply electrode 12a and second current supply electrode 12b in handheld unit 10. In the present embodiment, the frequency of alternating current output from first electric current generator 28 is set to 50 kHz (this is the same for an alternating current output from second electric current generator 32 described below). First voltage measurer 30 is a means for measuring voltage between first voltage detection electrode 12c and second voltage detection electrode 12d. Second electric current generator 32 is a means for outputting an alternating current that flows between third current supply electrode 23a and fourth current supply electrode 23b. Second voltage measurer 34 is a means for measuring voltage between third voltage detection electrode 23c and fourth voltage detection electrode 23d. Weight measurer 36 is a means for measuring the weight of a human subject who has stepped on platform unit 20 and for outputting weight data. Power source unit 38 is a means for supplying electricity to each part of the electrical system of platform unit 20.

Memory 42 is a means for storing various computation formulae for calculating body fat percentage, body fat mass, subcutaneous fat thickness, visceral fat area, visceral fat mass, subcutaneous fat area, and subcutaneous fat mass of a human subject, and body specific information (sex, height, age, etc.) input by input unit 26 and result information, etc. Controller 44 is a means for executing various control processes.

B: Operation of subcutaneous fat thickness measurement apparatus

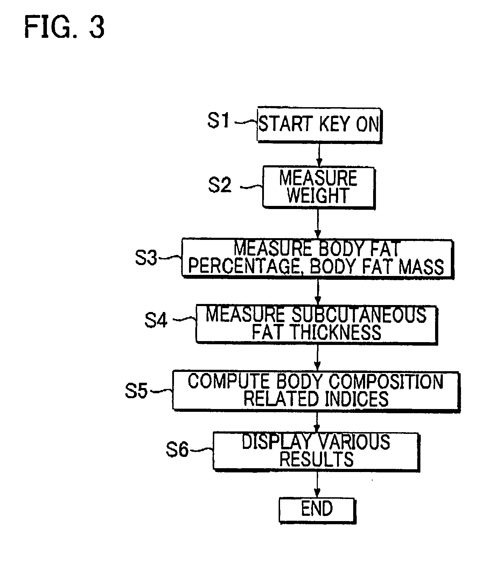

Description will next be given of operation of subcutaneous fat thickness measurement apparatus 100. In the present embodiment, a human subject first holds handheld unit 10 and steps barefoot on second measurement electrodes 23 of platform unit 20. The human subject then presses the forefront portion of handheld unit 10 onto a portion of his or her body of which subcutaneous fat thickness is to be measured. Various measurement results (subcutaneous fat thickness, etc.) are displayed on display unit 22. In the following, reference is made to Fig. 3 to describe the details of the operation. Fig. 3 is a flowchart showing a procedure of a detailed operation of subcutaneous fat thickness measurement apparatus 100 according to the present embodiment.

In a case in which start key 26d is first turned on by a human subject (Step S1), power supply from power source unit 38 is started, which in turn causes subcutaneous fat thickness measurement apparatus 100 to change to a measurement mode, In a case in which setting key 26a is changed to an on-state when start key 26d has not been turned on (i.e., when the power is off), the apparatus enters a setting mode in which body specific information can be set. In this mode, a cursor appears at one of sex, height, and age displayed on display unit 22, and a human subject can set these pieces of information or change numerals by operating UP key 26b, DOWN key 26c, and setting key 26a. Body specific information set in this way is stored in memory 42. In a case in which body specific information was not set in the past, the information will be newly registered; and in a case in which body specific information was set in the past, the information will be renewed.

When the human subject then steps on platform unit 20, controller 44 measures the weight of the human subject (Step S2).

More specifically, weight measurer 36 outputs weight data corresponding to the weight of the human subject when the human subject steps on platform unit 20. Controller 44 obtains the weight of a human subject based on weight data output from weight measurer 36, and stores the value thereof into memory 42.

Controller 44 then measures body fat percentage and body fat mass of the human subject (Step S3). More specific description follows. The sole of the left foot of the human subject is now in touch with third current supply electrode 23a and with third voltage detection electrode 23c. Furthermore, the sole of the right foot is now in touch with of the fourth current supply electrode 23b and with the fourth voltage detection electrode 23d. As a result, a current pathway is formed, the pathway starting from one electrode of third current supply electrode 23a and fourth current supply electrode 23b via the human subject to the other electrode of third current supply electrode 23a and fourth current supply electrode 23b. An alternating current output from second electric current generator 32 flows in this current pathway. Controller 44 obtains foot-to-foot bioelectrical impedance of the human subject from the value of an electric current that flows in this current pathway and the value of voltage measured by second voltage measurer 34, to store the result in memory 42.

Controller 44 obtains body fat percentage by assigning weight, foot-to-foot bioelectrical impedance, sex, height, and age of the human subject into a computation formula stored in memory 42. The body fat percentage fp is expressed by the following formula (1).

$$fp = k1 * Zle50 + k2 * weight + k3 * height + k4 * age + k5 * sex + k6 \ .....(1),$$

wherein fp is body fat percentage, Zle50 is foot-to-foot bioelectrical impedance, and k1 to k6 are constants.

Furthermore, controller 44 obtains body fat mass by assigning body fat percentage fp obtained by the formula (1) and the weight of the human subject into a computation formula for body fat mass stored in memory 42, to obtain body fat mass. The computation formula of body fat mass is expressed by the following formula (2).

$$fa = fp * weight \ ..... (2),$$

wherein fa is body fat mass.

Thus, controller 44 obtains weight, as obesity-related information, measured by weight measure 36 and also obtains body fat percentage fp and body fat mass fa, as obesity-related information, based on the measured weight, and foot-to-foot bioelectrical impedance obtained by using second measurement electrodes 23, second electric current generator 32, and second voltage measure 34. Therefore, controller 44, weight measurer 36, second measurement electrodes 23, second electric current generator 32, and second voltage measurer 34 serve as an obesity information measurer for measuring obesity-related information in cooperation with one another,

Subsequently, when the human subject presses the forefront face of handheld unit 10 onto a portion of the human subject, subcutaneous fat thickness of which the human subject wishes to measure, controller 44 measures subcutaneous fat thickness of a portion with which first measurement electrodes 12 (12a,12b,12c,12d) are in contact, the portion being a portion of the body of the human subject (Step S4). More specifically, when first measurement electrodes 12 (12a,12b,12c,12d) come into contact with a human subject, a current pathway starting from the electrode of one of first current supply electrode 12a and second current supply electrode 12b via the human subject reaching the electrode of the other is formed. Alternating current output from first electric current generator 28 flows in this current pathway. Controller 44 obtains, based on the phase difference between the current that flows in this current pathway and voltage measured by first voltage measurer 30, subcutaneous fat thickness between first current supply electrode 12a and second current supply electrode 12b.

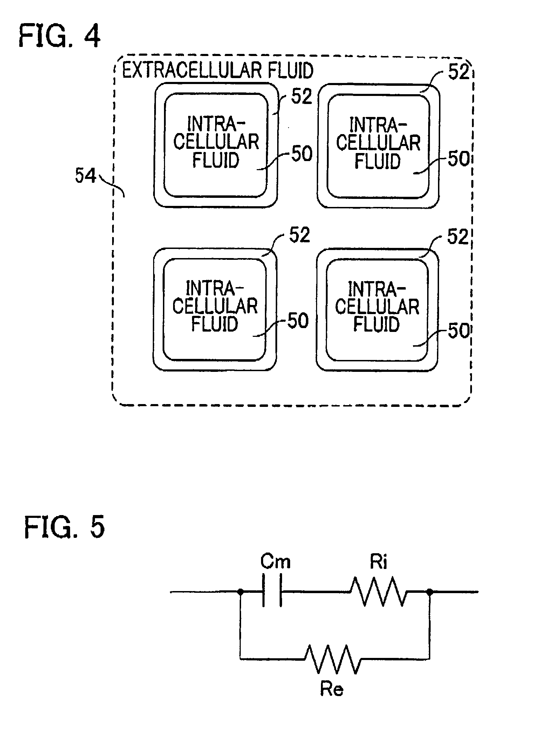

A detailed description will now be given of the difference between the phase difference caused when alternating current output from first electric current generator 28 flows through the muscle layer of a human subject and the phase difference caused when the alternating current flows through the fat layer. As shown in Fig. 4, the tissue of a human body (muscular tissue and fat tissue) has plural cell membranes 52 each having intracellular fluid 50 and extracellular fluid 54 mediated between cell membranes 52. Given that the capacitance of cell membrane 52 is Cm, that resistance of intracellular fluid 50 is Ri, and that the resistance of extracellular fluid 54 is Re, the muscular tissue and fat tissue can be expressed by an equivalent circuit shown in Fig. 5.

Because cell membrane 52 of the fat tissue contains little intracellular fluid 50, the value of resistance Ri of intracellular fluid 50 will be an extremely large value in comparison with the value of resistance Re of extracellular fluid 54 (Re << Ri). For this reason, when alternating current output from first electric current generator 28 flows through the fat tissue, most of the current flows through resistance Re of extracellular fluid. In this case, the phase difference between this current and voltage measured by first voltage measurer 30 is not likely to be caused. In contrast, cell membrane 52 of the muscular tissue contains intracellular fluid 50. Therefore, when alternating current output from first electric current generator 28 flows through the muscular tissue, the current flows not only through the resistance Re of extracellular fluid but also through the capacitance Cm of cell membrane 52 and the resistance Ri of intracellular fluid 50. That is, when alternating current is output from first electric current generator 28, the phase difference is caused between the current and the voltage measured by first voltage measurer 30.

Thus, the muscle layer has a property that easily causes the phase difference, whereas the fat layer has a property that is difficult to cause the phase difference. Therefore, the greater the subcutaneous fat thickness, the more dominant the property of the fat layer, and the smaller the phase difference, whereas the smaller the subcutaneous fat thickness, the more dominant the property of the muscle layer, and the greater the phase difference. In the present embodiment, this is used to measure subcutaneous fat thickness.

More specific description will follow. Controller 44 obtains the phase difference and also calculates impedance based on the current output from first electric current generator 28 and the voltage measured by first voltage measurer 30 when first measurement electrodes 12 (12a,12b,12c,12d) are in contact with a human body. The obtained phase difference is that which is between the first current supply electrode and the second current supply electrode. Furthermore, controller 44, based on the phase difference and the impedance, obtains resistance R that is the real part of the impedance and reactance X that is the imaginary part of the impedance, to obtain the ratio between reactance X and resistance R, R/X. In a case in which the phase difference is smaller, the proportion of reactance X in resistance R becomes smaller; whereas in a case in which the phase difference is greater, the proportion of reactance X in resistance R becomes greater. Controller 44 determines subcutaneous fat thickness corresponding to the obtained R/X.

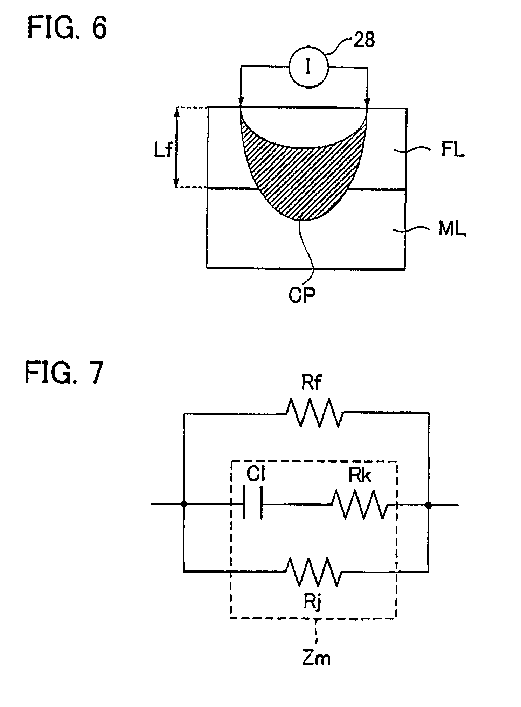

Description will now be given of the relationship between the above R/X and the subcutaneous fat thickness. Fig. 6 is a diagram schematically describing how alternating current output from first electric current generator 28 flows through the fat layer FL and the muscle layer ML of a human subject when first measurement electrodes 12 are in touch with a human subject. The shaded portion shown in Fig. 6 shows the current pathway CP of alternating current. Furthermore, Lf shown in Fig. 6 shows the thickness of the fat layer FL (i.e., subcutaneous fat thickness). Fig. 7 is a diagram showing an equivalent circuit of the fat layer and the muscle layer corresponding to the diagram shown in Fig. 6. Rf shown in Fig. 7 indicates the resistance of the fat layer. As described above, in the fat layer, the capacitance can be almost ignored. Furthermore, an area designated by Zm shows a portion corresponding to the muscle layer; Rj indicates the resistance of the extracellular fluid 54 of the muscle layer; Rk indicates the resistance of intracellular fluid 50 of the muscle layer; and Cl indicates the capacitance of cell membrane 52 of the muscle layer. In this case, the ratio R/X between reactance X and resistance R of a portion with which the measurement electrodes are in contact, from among the body of a human subject, is expressed by the following formula (3).

$$R/X = -\omega C1Rk - \{(\omega C1Rk)^2 + 1\}/(\omega C1Rj) - \{(\omega C1Rk)^2 + 1\}/(\omega C1Rf) \,\ldots\, (3)$$

Furthermore, because the resistance Rf of fat layer is inversely proportional to subcutaneous fat thickness Lf, the relationship therebetween is expressed by the following formula (4).

$$Rf = k/Lf \,\ldots\, (4),$$

wherein k is a constant.

Given the above formula (3) and formula (4), subcutaneous fat thickness Lf can be expressed by the following formula (5).

$$Lf = -\omega C1k/\{(\omega C1Rk)^2 + 1\}*[(\omega C1Rk)+\{(\omega C1Rk)^2 + 1\}/(\omega C1Rj) + R/X]$$

$$= -a - b * R/X \,\ldots\, (5),$$

wherein a and b are constants. As is understood from the above formula (5), the subcutaneous fat thickness Lf and R/X are in a proportional relationship. That is, as subcutaneous fat thickness Lf becomes greater, the property of the fat layer becomes more dominant, and the phase difference becomes smaller. Therefore, the proportion of reactance X in resistance R becomes smaller (the value of R/X becomes greater). On the other hand, as subcutaneous fat thickness Lf becomes smaller, the property of the muscle layer becomes more dominant, and the phase difference becomes larger. Therefore, the proportion of reactance X in resistance R becomes greater (the value of R/X becomes smaller).

In the present embodiment, the above formula (5) is stored in memory 42 in advance. Controller 44 assigns the value of RIX obtained earlier into the computation formula for subcutaneous fat thickness (the above-described formula (5)) stored in memory 42, to determine the value of subcutaneous fat thickness Lf.

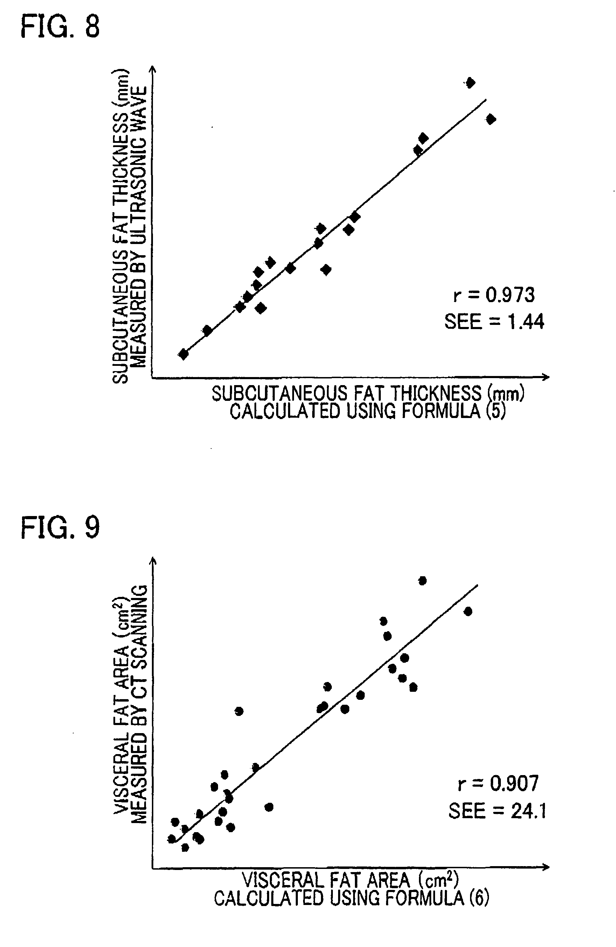

Fig. 8 is a correlation diagram showing the calculated value of subcutaneous fat thickness obtained by using the above formula (5) and the actual measured value of subcutaneous fat thickness measured by the ultrasonic wave. As shown in Fig. 8, the calculated values of subcutaneous fat thickness obtained by using the above formula (5) correlate highly with the actual values of subcutaneous fat thickness measured by the ultrasonic wave as indicated by a correlation coefficient r = 0.973. Furthermore, the standard accidental error estimate SEE (standard error estimation) is 1.44 mm, which means that the calculated values using the above formula (5) are within the range of accidental error 1.44 mm. Therefore, the calculated values have a high correlation with the actual values of subcutaneous fat thickness measured by the ultrasonic wave. Thus, according to the estimation method using the above formula (5), subcutaneous fat thickness can be estimated with a high degree of accuracy by using the value of R/X as a parameter.

Controller 44 then executes the calculation of body composition related indices of a human subject (Step S5 in Fig. 3). In the present embodiment, as "body composition related indices", visceral fat area, visceral fat mass, subcutaneous fat area, and subcutaneous fat mass are calculated. Detailed description will follow.

Controller 44 assigns each of subcutaneous fat thickness Lf and body fat mass fa of the human subject obtained earlier in a computation formula for visceral fat area stored in memory 42, thereby to obtain visceral fat area. The computation formula for visceral fat area can be expressed by the following formula (6).

$$\text{visceral fat area} = -c + (d * fa) + (e * Lf) \,\ldots\, (6),$$

where letters c to e are constants.

Fig. 9 is a correlation diagram showing a relationship between the calculated values of visceral fat area obtained by using the formula (6), and the values of visceral fat area by using a CT (Computed Tomography) scanning method which is considered as being capable of yielding highly accurate results of calculation. In the present embodiment, as shown in Fig. 9, the calculated values of visceral fat area obtained by using the above formula (6) highly correlates with the values of visceral fat area obtained by the CT scanning as indicated by the correlation coefficient r = 0.907. Furthermore, the standard accidental error estimate SEE is 24, $cm^2$, which means that the calculated values using the above formula (6) are within the range of accidental error 24.1 $cm^2$. Therefore, the calculated values have a high correlation with visceral fat area measured by the CT scanning. Therefore, according to an estimation method using the above formula (6), highly accurate estimation of visceral fat area is possible by using the values of subcutaneous fat thickness Lf and body fat mass fa as parameters.

Furthermore, controller 44 assigns each of subcutaneous fat thickness Lf and body fat mass fa of the human subject and the height of the human subject stored in memory 42, in a computation formula for visceral fat mass stored in memory 42, thereby to obtain visceral fat mass. The computation formula for visceral fat mass can be expressed by the following formula (7).

$$\text{visceral fat mass} = f + (g * fa) + (h * height) - (i * Lf) \quad \ldots\ldots (7),$$

where letters f to i are constants.

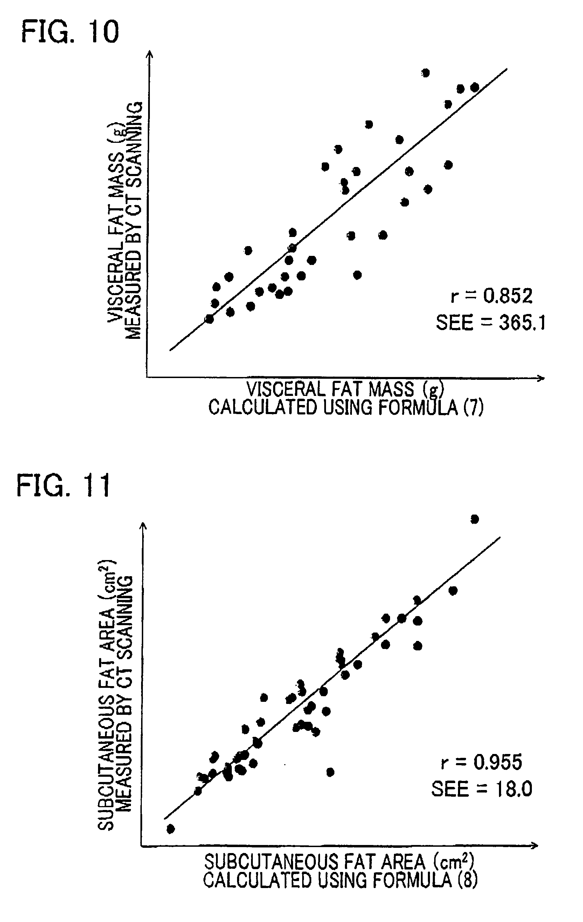

Fig. 10 is a correlation diagram showing the calculated values of visceral fat mass obtained by using the above formula (7) and the values of visceral fat mass measured by the CT scanning. In the present embodiment, as shown in Fig. 10, the calculated values of visceral fat mass obtained by using the above formula (7) highly correlate with the values of visceral fat mass measured by the CT scanning, as indicated by correlation coefficient r = 0.852. Furthermore, the standard accidental error estimate SEE is 365.1 g, which means that the calculated values obtained by using the above formula (7) are within the range of accidental error 365.1 g. Therefore, the calculated values are highly correlated with the values of visceral fat mass measured by the CT scanning, Therefore, according to an estimation method using the above formula (7), it is possible to perform a highly accurate estimation of visceral fat mass by using the values of subcutaneous fat thickness Lf, body fat mass fa, and the value of the height as parameters.

Furthermore, controller 44 assigns each of subcutaneous fat thickness Lf and body fat mass fa of a human subject in a computation formula for subcutaneous fat area stored in memory 42, thereby to obtain subcutaneous fat area. The computation formula for subcutaneous fat area can be expressed by the following formula (8).

$$\text{subcutaneous fat area} = j + (k * fa) + (l * Lf) \quad \ldots\ldots (8),$$

wherein letters j to l are constants.

Fig. 11 is a correlation diagram showing the relationship between the calculated values of subcutaneous fat area obtained by using the above formula (8) and the values of subcutaneous fat area measured by the CT scanning. In the present embodiment, as shown in Fig. 11, the calculated values of subcutaneous fat area obtained by using the above formula (8) highly correlate with the values of subcutaneous fat area measured by the CT scanning, as indicated by correlation coefficient r = 0.955.

Furthermore, the standard accidental error estimate SEE is 18.0 $cm^2$, which means that calculated values obtained by using the above formula (8) are within the range of accidental error 18.0 $cm^2$. Therefore, the calculated values are highly correlated with the values of subcutaneous fat area measured by the CT scanning. Therefore, according to an estimation method using the above formula (8), it is possible to perform a highly accurate estimation of subcutaneous fat area by using the values of subcutaneous fat thickness Lf and body fat mass fa as parameters.

Furthermore, controller 44 assigns each of subcutaneous fat thickness Lf, body fat mass fa, and height of a human subject into a computation formula for subcutaneous fat mass stored in memory 42, to obtain subcutaneous fat mass. The computation formula for subcutaneous fat mass can be expressed by the following formula (9).

$$\text{subcutaneous fat mass} = m + (n * fa) + (o * height) + (p * Lf) \quad \ldots\ldots$$
$$(9),$$

wherein letters m to p are constants.

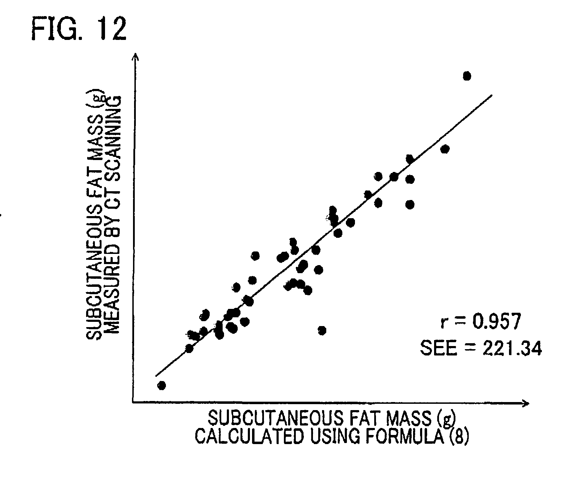

Fig. 12 is a correlation diagram showing the calculated values of subcutaneous fat mass obtained by using the above formula (9) and the values of subcutaneous fat mass measured by the CT scanning. In the present embodiment, as shown in Fig. 12, the calculated values of subcutaneous fat mass obtained by using the above formula (9) highly correlate with the values of subcutaneous fat mass measured by the CT scanning, as indicated by the correlation coefficient r = 0.957. Furthermore, the standard accidental error estimate SEE is 221.34 g, which means that calculated values obtained by using the above formula (9) are within the range of accidental error 221.34 g. Therefore, the calculated values obtained by using the above formula (9) are highly correlated with subcutaneous fat mass measured by the CT scanning. Therefore, according to an estimation method using the above formula (9), it is possible to perform a highly accurate estimation of subcutaneous fat mass by using the values of subcutaneous fat thickness Lf, body fat mass fa, and height as parameters.

Thus, controller 44 serves as a body composition related index measurer for measuring, as body composition related indices, visceral fat area, visceral fat mass, subcutaneous fat area, and subcutaneous fat mass of the human subject based on subcutaneous fat thickness of a human subject measured by the subcutaneous fat thickness obtainer and the obesity-related information of the human subject measured by the obesity information measurer.

When the process in Step S5 of Fig. 3 ends, controller 44 controls display unit 22 to display various results obtained in such ways as above (body fat percentage fp, body fat mass fa, subcutaneous fat thickness Lf, visceral fat area, visceral fat mass, subcutaneous fat area, and subcutaneous fat mass) (Step S6 in Fig. 3). As a result, a series of operations is completed.

As described in the foregoing, in the present embodiment, the focus is on the difference between the fat layer and the muscle layer in the phase difference that is generated when first measurement electrodes 12 (12a,12b,12c,12d) are in touch with a human body. The subcutaneous fat thickness Lf lying between first current supply electrode 12a and second current supply electrode 12b is then obtained based on the phase difference between a current that flows in a current pathway starting from one electrode of first current supply electrode 12a and second current supply electrode 12b via a human body to the other electrode and voltage measured by first voltage measurer 30.

More specifically, the muscle layer has a property that easily causes the phase difference, and the fat layer has a property that is difficult to cause the phase difference. Therefore, when the subcutaneous fat thickness Lf is greater, the property of the fat layer becomes more dominant, and the phase difference becomes smaller. In contrast, when subcutaneous fat thickness Lf is smaller, the property of the muscle layer becomes dominant, and the phase difference becomes greater. The greater the subcutaneous fat thickness Lf, the smaller the proportion of resistance R in reactance X, and the smaller subcutaneous fat thickness, the greater the proportion of reactance X in resistance R. Using this relationship, in the present embodiment, the ratio of resistance R to reactance X (R/X) is obtained, and the obtained value of the ratio is assigned in the above formula (5), thereby allowing determination of the corresponding subcutaneous fat thickness Lf. According to this embodiment, there is an advantage in that subcutaneous fat thickness Lf can be measured with a high degree of accuracy.

Furthermore, in the present embodiment, first current supply electrode 12a and second current supply electrode 12b are located sandwiched between first voltage detection electrode 12c and second voltage detection electrode 12d. Therefore, in comparison with a case in which first voltage detection electrode 12c and second voltage detection electrode 12d are sandwiched between first current supply electrode 12a and second current supply electrode 12b (hereinafter called "comparison example"), the distance L between first current supply electrode 12a and second current supply electrode 12d can be reduced. Generally, the amount of fat varies depending on the part of the human body (i.e., subcutaneous fat thickness Lf are different). Therefore, in a case in which the distance L between first current supply electrode 12a and second current supply electrode 12b used for measurement by contact with a human body is large, inadvertent measurement errors are likely to occur. According to the present embodiment, the distance L between first current supply electrode 12a and second current supply electrode 12d can be reduced in comparison with the comparison example, and subcutaneous fat thickness Lf can be measured with pinpoint localization. Therefore, in comparison with the comparison example, subcutaneous fat thickness Lf determination can be improved.

C: Modification

The present invention is not limited to the above-described embodiments. For example, the following modifications are possible. Furthermore, from among the Modification shown below, two or more modifications can be combined.

(1) Modification 1

In the above embodiment, subcutaneous fat thickness measurement apparatus 100 is provided, in addition to a function of measuring subcutaneous fat thickness Lf of a human subject, with a function of measuring obesity-related information, such as weight, body fat percentage fp, and body fat mass fa of a human subject, and a function of, by using the result of the measurements, measuring body composition related indices of a human subject (visceral fat area, visceral fat mass, subcutaneous fat area, and subcutaneous fat mass). However, subcutaneous fat thickness measurement apparatus 100 according to the present invention is not limited to the above embodiment. For example, it can be provided with only a function of measuring subcutaneous fat thickness Lf.

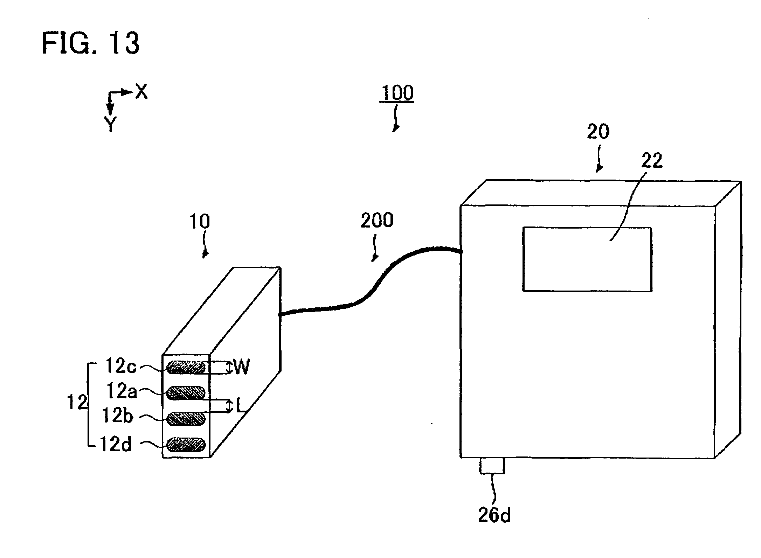

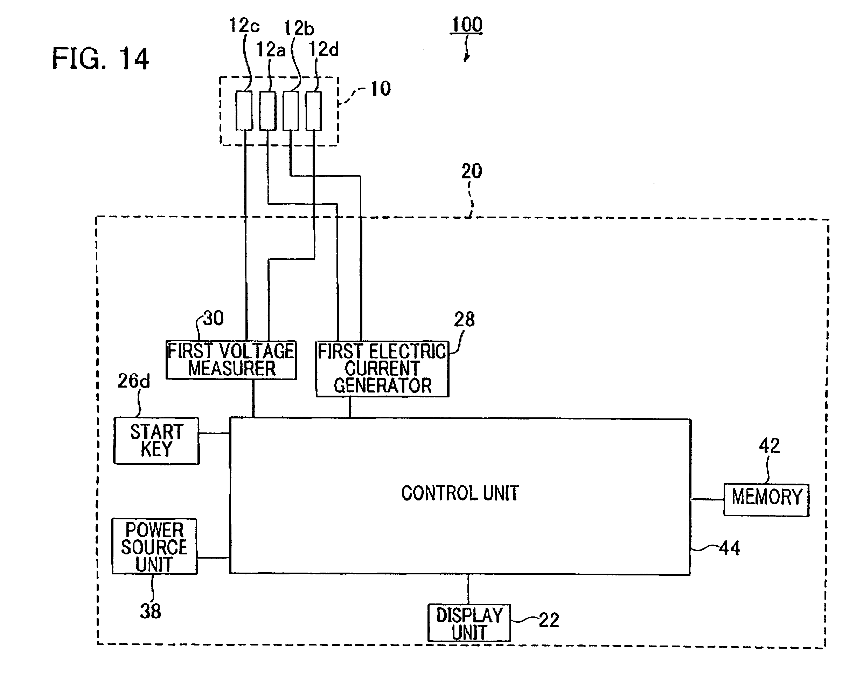

Fig. 13 is a diagram showing an external view of subcutaneous fat thickness measurement apparatus 100 which is provided only with a function of measuring subcutaneous fat thickness Lf, and Fig. 14 is a block diagram showing a detailed configuration of this subcutaneous fat thickness measurement apparatus 100. In Figs. 13 and 14, the configuration of handheld unit 10 is the same as the above embodiment. Platform unit 20 has a display unit 22, a start key 26d, a first electric current generator 28, a first voltage measurer 30, a power source unit 38, a memory 42, and a controller 44, but does not have means required for obtaining body fat percentage and the weight of a human subject such as second measurement electrodes 23 (23a,23b,23c,23d) and weight measurer 36. Similarly, controller 44 does not have a function required for obtaining body fat percentage or the weight of a human subject. Handheld unit 10 may be provided with the configuration of platform unit 20, so that handheld unit 10 and platform unit 20 are unified.

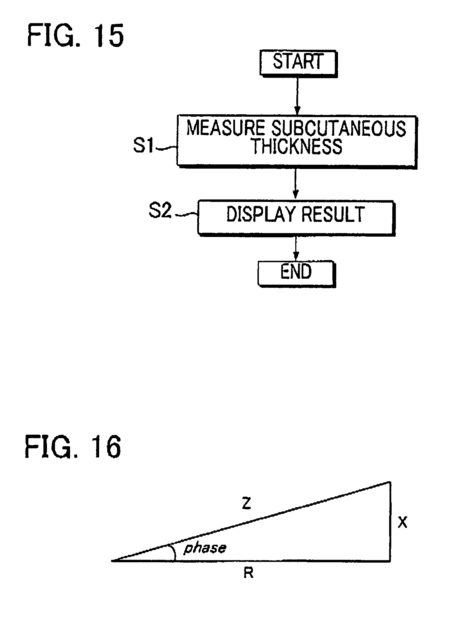

Fig. 15 is a flowchart showing the procedure of an operation performed by subcutaneous fat thickness measurement apparatus 100 provided with only a function of measuring subcutaneous fat thickness Lf. With reference to Fig. 15, description will be given of an operation of subcutaneous fat thickness measurement apparatus 100 provided with only a function of measuring subcutaneous fat thickness Lf. When start key 26d is first turned on by a human subject, power supply from power source unit 38 is started, and subcutaneous fat thickness measurement apparatus 100 then changes to an operable state. Then, when the human subject presses the forefront portion of handheld unit 10 to a portion of the body of the human subject, subcutaneous fat thickness Lf of which is to be measured, controller 44 measures subcutaneous fat thickness Lf between first current supply electrode 12a and second current supply electrode 12b using the same method as in the above-described embodiment (Step S1). When the process of Step S1 ends, controller 44 controls display unit 22 to display subcutaneous fat thickness Lf obtained in Step S1 (Step S2). As a result, the series of operations is completed.

(2) Modification 2

In the above embodiment, an example was given in which first current supply electrode 12a and second current supply electrode 12b are sandwiched between first voltage detection electrode 12c and second voltage detection electrode 12d, but this is not limited thereto. For example, first voltage detection electrode 12c and second voltage detection electrode 12d may be sandwiched between first current supply electrode 12a and second current supply electrode 12b.

(3) Modification 3

In the above embodiment, the distance L in the Y direction between first current supply electrode 12a and second current supply electrode 12b is set as being 5 mm, but this is not limited thereto. This distance L can be freely set within a range between 2 mm to 20 mm, inclusive. In short, this distance L can be any value so long as subcutaneous fat thickness Lf can be measured with a high degree of accuracy.

Furthermore, each of the distance in the Y direction between first current supply electrode 12a and first voltage detection electrode 12c and the distance in the Y direction between second current supply electrode 12b and second voltage detection electrode 12d is set to 5 mm in the above embodiment, but this is not limited thereto. This distance can be freely set within a range between 2 mm to 30 mm, inclusive. In short, the distance between the current supply electrode and the voltage detection electrode in the Y direction may be any value so long as the impedance of a portion of a human body that is in contact with measurement electrodes can be measured with a high degree of accuracy.

(4) Modification 4

In the above embodiments, reactance X and resistance R are used as bases for obtaining subcutaneous fat thickness Lf; however, the present invention is not limited thereto. Fig. 16 is a diagram showing the relationship among impedance, reactance X, resistance R, and the phase difference. In Fig. 16, the impedance is denoted as Z, and the phase difference as "*phase*",

As understand from Fig. 16, because $R = (Z^2 - X^2)^{1/2}$ is true, R/X in the above formula (S) can be transformed into $\{(Z/X)^2 - 1\}^{1/2}$. Therefore, the subcutaneous fat thickness Lf may be determined based on the value of Z/X.

Additionally, as understood from Fig. 16, because $X = (Z^2 - R^2)^{1/2}$ is true, R/X in the above formula (5) can be transformed into $1/\{(Z/R)^2-1\}^{1/2}$. Therefore, the subcutaneous fat thickness Lf may be determined based on the value of Z/R.

Thus, the subcutaneous fat thickness Lf can be determined based on impedance Z and reactance X; or the subcutaneous fat thickness Lf can be determined based on impedance Z and resistance R.

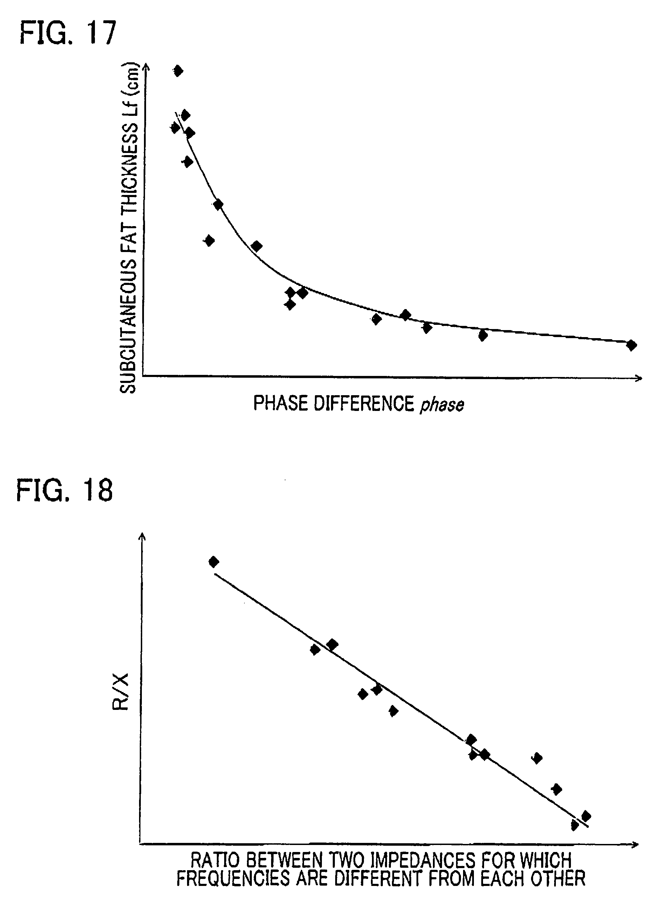

The phase difference can be expressed as *phase* = *arctan* (R/X). In taking a measurement of a human body, the value of impedance Z is almost equal to resistance R. Therefore, the phase difference can be expressed also as *phase* ≈ (approximately equal to) *arctan* (Z/X). Fig. 17 is a diagram showing the relationship between the phase difference *phase* and the subcutaneous fat thickness Lf. Thus, the subcutaneous fat thickness Lf can be obtained only based on the phase difference *phase*.

In another alternative, the subcutaneous fat thickness Lf may be obtained by using the values of two impedances for which frequencies are different from each other. As the frequency is changed in measuring bioelectric impedance, the plotting of the values of impedance Z gives a circular arc publicly known as Cole-Cole plot. Therefore, obtaining two values of impedance Z enables the estimation of the size of the circular arc (i.e, the coordinates of its center, diameter, etc.) of the Cole-Cole plot. Each value of impedance Z, reactance X, resistance R, and the phase difference *phase* changes if the frequency changes, whereas R/X stays constant even if the frequency changes.

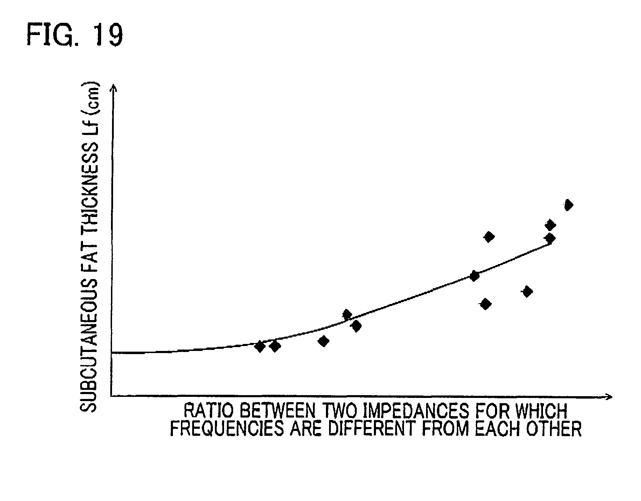

Fig. 18 is a diagram showing the relationship between the ratio between two impedances Z for which frequencies are different from each other and R/X. An example of the ratio between two impedances Z for which frequencies are different from each other shown in Fig, 18 is the ratio (Z250/Z50) between the value of impedance Z250 obtained by using the frequency of 250 kHz and the value of impedance Z50 obtained by using the frequency of 50 kHz. Thus, Fig. 18 shows the relationship between Z250/Z50 and R/X. Fig. 19 shows the relationship between the ratio between two impedances Z for which frequencies are different from each other (Z250/Z50 in this embodiment) and the subcutaneous fat thickness Lf. As understood from Figs. 18 and 19, the subcutaneous fat thickness Lf can be obtained based on two impedances Z for which frequencies are different from each other.

(5) Modification 5

In the above embodiment, controller 44 measures, as body composition related indices, visceral fat area, visceral fat mass, subcutaneous fat area, and subcutaneous fat mass of the human subject. Alternatively, controller 44 may measure at least one of visceral fat area, visceral fat mass, subcutaneous fat area, or subcutaneous fat mass of the human subject. In this case, a human subject may select at least one desired body composition related index by using input unit 26 so that only the desired body composition related index is measured by subcutaneous fat thickness measurement apparatus 100. In another alternative, a human subject may select no body composition related index to be measured. In this case, subcutaneous fat thickness measurement apparatus 100 only measures subcutaneous fat thickness Lf.

(6) Modification 6

In the above embodiment, a human subject uses subcutaneous fat thickness measurement apparatus 100, but a person other than the human subject (for example, a caregiver of a human subject) may use subcutaneous fat thickness measurement apparatus 100 to measure or obtain subcutaneous fat thickness and other indices such as obesity-related information and body composition related indices of the human subject.

**Claims**

1. A subcutaneous fat thickness measurement apparatus (100) comprising:

   measurement electrodes (12) including a first current supply electrode (12a), a second current supply electrode (12b), a first voltage detection electrode (12c), and a second voltage detection electrode (12d), for use in measurement by contact with the body of a human subject;
   an electric current generator (28) for outputting alternating current that flows between the first current supply electrode and the second current supply electrode;
   a voltage measurer (30) for measuring voltage between the first voltage detection electrode and the second voltage detection electrode, the first voltage detection electrode located adjacent to the first current supply electrode and the second voltage detection electrode located adjacent to the second current supply electrode; and
   a subcutaneous fat thickness measurer (44) for obtaining subcutaneous fat thickness of a portion of the body between the first current supply electrode and the second current supply electrode that are in contact with the body based on the phase difference between electric current and voltage, the electric current flowing in a current pathway from one of the first current supply electrode and the second current supply electrode via the human body to the other of the first current supply electrode and the second current supply electrode when the measurement electrodes are in contact with the human body and the voltage being measured by the voltage measurer, **characterized in that**:

   the subcutaneous fat thickness measurer is adapted to obtain a ratio between reactance and resistance obtained from impedance and the phase difference, to determine subcutaneous fat thickness corresponding to the obtained value of ratio, the impedance being calculated from the electric current flowing in the current pathway and the voltage measured by the voltage measurer.

2. A subcutaneous fat thickness measurement apparatus (100) according to claim 1, wherein; subcutaneous fat thickness obtained in the subcutaneous fat thickness measurer results in a greater value as a proportion of the reactance in the resistance is smaller, and in a smaller value as the proportion of the reactance in the resistance is greater.

3. A subcutaneous fat thickness measurement apparatus (100) comprising:

measurement electrodes (12) including a first current supply electrode (12a);
a second current supply electrode (12b), a first voltage detection electrode (12c) and a second voltage detection electrode (12d), for use in measurement by contact with the body of a human subject;
an electric current generator (28) for outputting alternating current that flows between the first current supply electrode and the second current supply electrode;
a voltage measurer (30) for measuring voltage between the first voltage detection electrode and the second voltage detection electrode, the first voltage detection electrode located adjacent to the first current supply electrode and the second voltage detection electrode located adjacent to the second current supply electrode; and
a subcutaneous fat thickness measurer (44) for obtaining subcutaneous fat thickness of a portion of the body between the first current supply electrode and the second current supply electrode that are in contact with the body, based on the phase difference between electric current and voltage, the electric current flowing in a current pathway from one of the first current supply electrode and the second current supply electrode via the human body to the other side of the first current supply electrode and the second current supply electrode when the measurement electrodes are in contact with the human body and the voltage being measured by the voltage measurer,
**characterized in that**:
the subcutaneous fat thickness measurer is adapted to obtain a ratio between impedance and reactance obtained from the impedance and the phase difference, to determine subcutaneous fat thickness corresponding to the obtained value of ratio, the impedance being calculated from the electric current flowing in the current pathway and the voltage measured by the voltage measurer.

4. A subcutaneous fat thickness measurement apparatus (100):

measurement electrodes (12) including a first current supply electrode (12a), a second current supply electrode (12b), a first voltage detection electrode (12c), and a second voltage detection electrode (12d), for use in measurement by contact with the body of a human subject;
an electric current generator (28) for outputting alternating current that flows between the first current supply electrode and the second current supply electrode;
a voltage measurer (30) for measuring voltage between the first voltage detection electrode and the second voltage detection electrode, the first voltage detection electrode located adjacent to the first current supply electrode and the second voltage detection electrode located adjacent to the second current supply electrode; and
a subcutaneous fat thickness measurer (44) for obtaining subcutaneous fat thickness of a portion of the body between the first current supply electrode and the second current supply electrode that are in contact with the body, based on the phase difference between electric current and voltage, the electric current flowing in a current pathway from one of the first current supply electrode and the second current supply electrode via the human body to the other of the first current supply electrode and the second current supply electrode when the measurement electrodes are in contact with the human body and the voltage being measured by the voltage measurer, **characterized in that**;
the subcutaneous fat thickness measurer is adapted to obtain a ratio between impedance and resistance obtained from the impedance and the phase difference, to determine subcutaneous fat thickness corresponding to the obtained value of ratio, the impedance being calculated from the electric current flowing in the current pathway and the voltage measured by the voltage measurer.

5. A subcutaneous fat thickness measurement apparatus (100) according to one of Claims 1 to 4, wherein; the first current supply electrode and the second current supply electrode are located sandwiched between the first voltage detection electrode and the second voltage detection electrode.

6.  A subcutaneous fat thickness measurement apparatus (100) according to Claim 5, wherein;
    the first current supply electrode, the second current supply electrode, the first voltage detection electrode, and the second voltage detection electrode are arranged along a first direction, and the distance (L) in the first direction between the first current supply electrode and the second current supply electrode is smaller than the sum of width (W) of the first voltage detection electrode in the first direction and width (W) of the second voltage detection electrode in the first direction.

7.  A subcutaneous fat thickness measurement apparatus (100), according to one of Claims 1 to 6, further comprising:

    an obesity information measurer (23, 32, 34, 36, 44) for measuring obesity-related information of the human subject; and
    a body composition related index measurer (44) for obtaining a body composition index of the human subject based on subcutaneous fat thickness of the human subject measured by the subcutaneous fat thickness measurer and the obesity-related information of the human subject measured by the obesity information measurer.

**Patentansprüche**

1.  Subkutane Fettdickenmessvorrichtung (100) die Folgendes umfasst:

    Messelektroden (12), die eine erste Stromversorgungselektrode (12a) beinhalten, eine zweite Stromversorgungselektrode (12b), eine erste Spannungsmesselektrode (12c) und eine zweite Spannungsmesselektrode (12d) zur Verwendung bei der Messung in Kontakt mit dem Körper eines Menschen,
    einen Stromgenerator (28) zur Produktion von Wechselstrom, der zwischen der ersten Stromversorgungselektrode und der zweiten Stromversorgungselektrode fließt,
    einen Spannungsmesser (30) zum Messen der Spannung zwischen der ersten Spannungsmesselektrode und der zweiten Spannungsmesselektrode, wobei sich die erste Spannungsmesselektrode neben der ersten Stromversorgungselektrode und die zweite Spannungsmesselektrode neben der zweiten Stromversorgungselektrode befindet, und
    eine subkutane Fettdickenmessvorrichtung (44) zur Ermittlung der subkutanen Fettdicke eines Körperbereichs zwischen der ersten Stromversorgungselektrode und der zweiten Stromversorgungselektrode, die mit dem Körper in Kontakt sind, was auf dem Phasenunterschied zwischen Strom und Spannung basiert, wobei der Strom in einem Strompfad von einer der ersten Stromversorgungselektrode und der zweiten Stromversorgungselektrode über den menschlichen Körper zur anderen der ersten Stromversorgungselektrode und der zweiten Stromversorgungselektrode fließt, wenn die Messelektroden mit dem menschlichen Körper in Kontakt sind, und die Spannung, die mit dem Spannungsmesser gemessen wird, **gekennzeichnet dadurch, dass**
    die subkutane Fettdickenmessvorrichtung so adaptiert wurde, dass ein Verhältnis zwischen Reaktanz und Widerstand, die von der Impedanz und der Phasendifferenz erhalten wurden, um die subkutane Fettdicke zu bestimmen, die mit dem erhaltenen Verhältniswert korrespondiert, wobei die Impedanz von dem im Strompfad fließenden Strom, und der Spannung, die vom Spannungsmesser gemessen wurde, berechnet wurde.

2.  Subkutane Fettdickenmessvorrichtung (100) nach Anspruch 1, wobei
    die subkutane Fettdicke, die mit der subkutanen Fettdickenmessvorrichtung erhalten wurde, einen höheren Wert ergibt, wenn ein Anteil der Reaktanz des Widerstands kleiner ist, und einen niedrigeren Wert, wenn ein Anteil der Reaktanz des Widerstands größer ist.

3.  Subkutane Fettdickenmessvorrichtung (100), die Folgendes umfasst:

    Messelektroden (12), die eine erste Stromversorgungselektrode (12a) beinhalten,
    eine zweite Stromversorgungselektrode (12b), eine erste Spannungsmesselektrode (12c) und eine zweite Spannungsmesselektrode (12d) zur Verwendung bei der Messung in Kontakt mit dem Körper eines Menschen,
    einen Stromgenerator (28) zur Produktion von Wechselstrom, der zwischen der ersten Stromversorgungselektrode und der zweiten Stromversorgungselektrode fließt,
    einen Spannungsmesser (30) zum Messen der Spannung zwischen der ersten Spannungsmesselektrode und der zweiten Spannungsmesselektrode, wobei sich die erste Spannungsmesselektrode neben der ersten Stromversorgungselektrode und die zweite Spannungsmesselektrode neben der zweiten Stromversorgungselektrode befindet, und

eine subkutane Fettdickenmessvorrichtung (44) zur Ermittlung der subkutanen Fettdicke eines Körperbereichs zwischen der ersten Stromversorgungselektrode und der zweiten Stromversorgungselektrode, die mit dem Körper in Kontakt sind, was auf dem Phasenunterschied zwischen Strom und Spannung basiert, wobei der Strom in einem Strompfad von einer der ersten Stromversorgungselektrode und der zweiten Stromversorgungselektrode über den menschlichen Körper zur anderen Seite der ersten Stromversorgungselektrode und der zweiten Stromversorgungselektrode fließt, wenn die Messelektroden mit dem menschlichen Körper in Kontakt sind, und die Spannung, die mit dem Spannungsmesser gemessen wurde, **gekennzeichnet dadurch, dass** die subkutane Fettdickenmessvorrichtung so adaptiert wurde, dass ein Verhältnis zwischen Reaktanz und Widerstand, die von der Impedanz und der Phasendifferenz erhalten wurden, um die subkutane Fettdicke zu bestimmen, die mit dem erhaltenen Verhältniswert korrespondiert, wobei die Impedanz von dem im Strompfad fließenden Strom, und der Spannung, die vom Spannungsmesser gemessen wurde, berechnet wurde.

4.  Subkutane Fettdickenmessvorrichtung (100):

Messelektroden (12), die eine erste Stromversorgungselektrode (12a) beinhalten, eine zweite Stromversorgungselektrode (12b), eine erste Spannungsmesselektrode (12c) und eine zweite Spannungsmesselektrode (12d) zur Verwendung bei der Messung in Kontakt mit dem Körper eines Menschen,

einen Stromgenerator (28) zur Produktion von Wechselstrom, der zwischen der ersten Stromversorgungselektrode und der zweiten Stromversorgungselektrode fließt,

einen Spannungsmesser (30) zum Messen der Spannung zwischen der ersten Spannungsmesselektrode und der zweiten Spannungsmesselektrode, wobei sich die erste Spannungsmesselektrode neben der ersten Stromversorgungselektrode und die zweite Spannungsmesselektrode neben der zweiten Stromversorgungselektrode befindet, und

eine subkutane Fettdickenmessvorrichtung (44) zur Ermittlung der subkutanen Fettdicke eines Körperbereichs zwischen der ersten Stromversorgungselektrode und der zweiten Stromversorgungselektrode, die mit dem Körper in Kontakt sind, was auf dem Phasenunterschied zwischen Strom und Spannung basiert, wobei der Strom in einem Strompfad von einer der ersten Stromversorgungselektrode und der zweiten Stromversorgungselektrode über den menschlichen Körper zur anderen der ersten Stromversorgungselektrode und der zweiten Stromversorgungselektrode fließt, wenn die Messelektroden mit dem menschlichen Körper in Kontakt sind, und die Spannung, die mit dem Spannungsmesser gemessen wird, **gekennzeichnet dadurch, dass** die subkutane Fettdickenmessvorrichtung so adaptiert wurde, dass ein Verhältnis zwischen Impedanz und Widerstand von der Impedanz und der Phasendifferenz erhalten wurden, um die subkutane Fettdicke zu bestimmen, die mit dem erhaltenen Verhältniswert korrespondiert, wobei die Impedanz von dem im Strompfad fließenden Strom, und der Spannung, die vom Spannungsmesser gemessen wurde, berechnet wurde.

5.  Subkutane Fettdickenmessvorrichtung (100) nach einem der Ansprüche 1 bis 4, wobei die erste Stromversorgungselektrode und die zweite Stromversorgungselektrode zwischen der ersten Spannungsmesselektrode und der zweiten Spannungsmesselektrode eingeklemmt sind.

6.  Subkutane Fettdickenmessvorrichtung (100) nach Anspruch 5, wobei die erste Stromversorgungselektrode, die zweite Stromversorgungselektrode, die erste Spannungsmesselektrode und die zweite Spannungsmesselektrode in einer ersten Richtung angeordnet sind, und die Distanz (L) in der ersten Richtung zwischen der ersten Stromversorgungselektrode und der zweiten Stromversorgungselektrode kleiner als die Summe der Breite (W) der ersten Spannungsmesselektrode in erster Richtung, und die Breite (W) der zweiten Spannungsmesselektrode in erster Richtung ist.

7.  Subkutane Fettdickenmessvorrichtung (100) nach einem der Ansprüche 1 bis 6, die ferner Folgendes umfasst:

einen Fettsucht-Informationsmesser (23, 32, 34, 36, 44) zum Messen der auf Fettsucht bezogenen Informationen über den Menschen, und

eine mit der Körperzusammensetzung zusammenhängender Indexmesser (44) zur Bestimmung der Körperzusammensetzung der Versuchsperson, die auf der subkutanen Fettdicke der Versuchperson basiert, der von der subkutanen Fettdickenmessvorrichtung und den auf die Fettsucht bezogenen Informationen der Versuchsperson, das vom Fettsucht-Informationsmesser erhalten wurde.

**Revendications**

1.  Appareil de mesure de l'épaisseur de la graisse du tissu sous-cutané (100) comportant :

    des électrodes de mesure (12) comprenant une première électrode d'alimentation en courant (12a), une seconde électrode d'alimentation en courant (12b), une première électrode de détection de tension (12c), et une seconde électrode de détection de tension (12d), à des fins d'utilisation pour la mesure par contact avec le corps d'un sujet humain ;
    un générateur de courant électrique (28) permettant de fournir un courant alternatif qui circule entre la première électrode d'alimentation en courant et la seconde électrode d'alimentation en courant ;
    un dispositif de mesure de tension (30) permettant de mesurer la tension entre la première électrode de détection de tension et la seconde électrode de détection de tension, la première électrode de détection de tension étant située de manière adjacente par rapport à la première électrode d'alimentation en courant et la seconde électrode de détection de tension étant située de manière adjacente par rapport à la seconde électrode d'alimentation en courant ; et
    un dispositif de mesure de l'épaisseur de la graisse du tissu sous-cutané (44) permettant d'obtenir l'épaisseur de la graisse du tissu sous-cutané d'une partie du corps entre la première électrode d'alimentation en courant et la seconde électrode d'alimentation en courant qui sont en contact avec le corps en se basant sur la différence de phase entre le courant électrique et la tension, le courant électrique circulant dans une voie de passage de courant allant de l'une parmi la première électrode d'alimentation en courant et la seconde électrode d'alimentation en courant par le biais du corps humain jusqu'à l'autre parmi la première électrode d'alimentation en courant et la seconde électrode d'alimentation en courant quand les électrodes de mesure sont en contact avec le corps humain et la tension en cours de mesure par le dispositif de mesure de tension, **caractérisé en ce que** :

    le dispositif de mesure de l'épaisseur de la graisse du tissu sous-cutané est adapté pour obtenir un rapport entre la réactance et la résistance que l'on obtient à partir de l'impédance et la différence de phase, pour déterminer l'épaisseur de la graisse du tissu sous-cutané correspondant à la valeur obtenue pour le rapport, l'impédance étant calculée à partir du courant électrique circulant dans la voie de passage de courant et la tension mesurée par le dispositif de mesure de tension.

2.  Appareil de mesure de l'épaisseur de la graisse du tissu sous-cutané (100) selon la revendication 1, dans lequel :

    l'épaisseur de la graisse du tissu sous-cutané obtenue dans le dispositif de mesure de l'épaisseur de la graisse du tissu sous-cutané donne lieu à une valeur supérieure quand une proportion de la réactance dans la résistance est inférieure, et à une valeur inférieure quand la proportion de la réactance dans la résistance est supérieure.

3.  Appareil de mesure de l'épaisseur de la graisse du tissu sous-cutané (100) comportant :

    des électrodes de mesure (12) comprenant une première électrode d'alimentation en courant (12a) ;
    une seconde électrode d'alimentation en courant (12b), une première électrode de détection de tension (12c) et une seconde électrode de détection de tension (12d), à des fins d'utilisation pour la mesure par contact avec le corps d'un sujet humain ;
    un générateur de courant électrique (28) permettant de fournir un courant alternatif qui circule entre la première électrode d'alimentation en courant et la seconde électrode d'alimentation en courant ;
    un dispositif de mesure de tension (30) permettant de mesurer la tension entre la première électrode de détection de tension et la seconde électrode de détection de tension, la première électrode de détection de tension étant située de manière adjacente par rapport à la première électrode d'alimentation en courant et la seconde électrode de détection de tension étant située de manière adjacente par rapport à la seconde électrode d'alimentation en courant ; et
    un dispositif de mesure de l'épaisseur de la graisse du tissu sous-cutané (44) permettant d'obtenir l'épaisseur de la graisse du tissu sous-cutané d'une partie du corps entre la première électrode d'alimentation en courant et la seconde électrode d'alimentation en courant qui sont en contact avec le corps, en se basant sur la différence de phase entre le courant électrique et la tension, le courant électrique circulant dans une voie de passage de courant allant de l'une parmi la première électrode d'alimentation en courant et la seconde électrode d'alimentation en courant par le biais du corps humain jusqu'à l'autre parmi la première électrode d'alimentation en courant et la seconde électrode d'alimentation en courant quand les électrodes de mesure sont en contact avec le corps humain et la tension en cours de mesure par le dispositif de mesure de tension, **caractérisé en ce que** :

le dispositif de mesure de l'épaisseur de la graisse du tissu sous-cutané est adapté pour obtenir un rapport entre l'impédance et la réactance que l'on obtient à partir de l'impédance et la différence de phase, pour déterminer l'épaisseur de la graisse du tissu sous-cutané correspondant à la valeur obtenue pour le rapport, l'impédance étant calculée à partir du courant électrique circulant dans la voie de passage de courant et la tension mesurée par le dispositif de mesure de tension.

4. Appareil de mesure de l'épaisseur de la graisse du tissu sous-cutané (100) :

des électrodes de mesure (12) comprenant une première électrode d'alimentation en courant (12a), une seconde électrode d'alimentation en courant (12b), une première électrode de détection de tension (12c), et une seconde électrode de détection de tension (12d), à des fins d'utilisation pour la mesure par contact avec le corps d'un sujet humain ;
un générateur de courant électrique (28) permettant de fournir un courant alternatif qui circule entre la première électrode d'alimentation en courant et la seconde électrode d'alimentation en courant ;
un dispositif de mesure de tension (30) permettant de mesurer la tension entre la première électrode de détection de tension et la seconde électrode de détection de tension, la première électrode de détection de tension étant située de manière adjacente par rapport à la première électrode d'alimentation en courant et la seconde électrode de détection de tension étant située de manière adjacente par rapport à la seconde électrode d'alimentation en courant ; et
un dispositif de mesure de l'épaisseur de la graisse du tissu sous-cutané (44) permettant d'obtenir l'épaisseur de la graisse du tissu sous-cutané d'une partie du corps entre la première électrode d'alimentation en courant et la seconde électrode d'alimentation en courant qui sont en contact avec le corps, en se basant sur la différence de phase entre le courant électrique et la tension, le courant électrique circulant dans une voie de passage de courant allant de l'une parmi la première électrode d'alimentation en courant et la seconde électrode d'alimentation en courant par le biais du corps humain jusqu'à l'autre parmi la première électrode d'alimentation en courant et la seconde électrode d'alimentation en courant quand les électrodes de mesure sont en contact avec le corps humain et la tension en cours de mesure par le dispositif de mesure de tension, **caractérisé en ce que** :

le dispositif de mesure de l'épaisseur de la graisse du tissu sous-cutané est adapté pour obtenir un rapport entre l'impédance et la résistance que l'on obtient à partir de l'impédance et la différence de phase, pour déterminer l'épaisseur de la graisse du tissu sous-cutané correspondant à la valeur obtenue pour le rapport, l'impédance étant calculée à partir du courant électrique circulant dans la voie de passage de courant et la tension mesurée par le dispositif de mesure de tension.

5. Appareil de mesure de l'épaisseur de la graisse du tissu sous-cutané (100) selon l'une quelconque des revendications 1 à 4, dans lequel :

la première électrode d'alimentation en courant et la seconde électrode d'alimentation en courant sont situées et prises en sandwich entre la première électrode de détection de tension et la seconde électrode de détection de tension.

6. Appareil de mesure de l'épaisseur de la graisse du tissu sous-cutané (100) selon la revendication 5, dans lequel :

la première électrode d'alimentation en courant, la seconde électrode d'alimentation en courant, la première électrode de détection de tension, et la seconde électrode de détection de tension sont arrangées le long d'une première direction, et la distance (L) dans la première direction entre la première électrode d'alimentation en courant et la seconde électrode d'alimentation en courant est inférieure à la somme de la largeur (W) de la première électrode de détection de tension dans la première direction et la largeur (W) de la seconde électrode de détection de tension dans la première direction.

7. Appareil de mesure de l'épaisseur de la graisse du tissu sous-cutané (100) selon l'une quelconque des revendications 1 à 6, comportant par ailleurs :

un dispositif de mesure d'informations d'obésité (23, 32, 34, 36, 44) permettant de mesurer des informations liées à l'obésité du sujet humain ; et
un dispositif de mesure d'indice lié à la composition corporelle (44) permettant d'obtenir un indice de composition corporelle du sujet humain en se basant sur l'épaisseur de la graisse du tissu sous-cutané du sujet humain mesurée par le dispositif de mesure de l'épaisseur de la graisse du tissu sous-cutané et sur les informations liées à l'obésité du sujet humain mesurées par le dispositif de mesure d'informations d'obésité.

FIG. 1

EP 2 238 900 B1

FIG. 2

EP 2 238 900 B1

# FIG. 3

S1 — START KEY ON

S2 — MEASURE WEIGHT

S3 — MEASURE BODY FAT PERCENTAGE, BODY FAT MASS

S4 — MEASURE SUBCUTANEOUS FAT THICKNESS

S5 — COMPUTE BODY COMPOSITION RELATED INDICES

S6 — DISPLAY VARIOUS RESULTS

END

# FIG. 4

# FIG. 5

FIG. 6

FIG. 7

## FIG. 8

## FIG. 9

## FIG. 10

r = 0.852
SEE = 365.1

VISCERAL FAT MASS (g)
MEASURED BY CT SCANNING

VISCERAL FAT MASS (g)
CALCULATED USING FORMULA (7)

## FIG. 11

r = 0.955
SEE = 18.0

SUBCUTANEOUS FAT AREA (cm²)
MEASURED BY CT SCANNING

SUBCUTANEOUS FAT AREA (cm²)
CALCULATED USING FORMULA (8)

FIG. 12

FIG. 13

EP 2 238 900 B1

FIG. 14

100

12c  12b
12a  12d

10

20

30
FIRST VOLTAGE
MEASURER

28
FIRST ELECTRIC
CURRENT
GENERATOR

26d
START
KEY

42
MEMORY

CONTROL UNIT

44

38
POWER
SOURCE
UNIT

22
DISPLAY
UNIT

EP 2 238 900 B1

FIG. 15

START

S1 — MEASURE SUBCUTANEOUS THICKNESS

S2 — DISPLAY RESULT

END

FIG. 16

Z

X

phase

R

## FIG. 17

## FIG. 18

FIG. 19

**EP 2 238 900 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2001178697 A
- US 2006100532 A